(19) Europäisches Patentamt
European Patent Office
Office européen des brevets

(11) Veröffentlichungsnummer : **0 125 591**
**B1**

(12) **EUROPÄISCHE PATENTSCHRIFT**

(45) Veröffentlichungstag der Patentschrift : **06.05.87**

(51) Int. Cl.[4] : **C 07 C119/048**, C 07 C118/02, A 01 N 47/00, C 08 G 18/76

(21) Anmeldenummer : **84105102.2**

(22) Anmeldetag : **05.05.84**

# (54) Trialkylsubstituierte aromatische Diisocyanate.

(30) Priorität : **14.05.83 DE 3317649**

(43) Veröffentlichungstag der Anmeldung : **21.11.84 Patentblatt 84/47**

(45) Bekanntmachung des Hinweises auf die Patenterteilung : **06.05.87 Patentblatt 87/19**

(84) Benannte Vertragsstaaten : **BE CH DE FR GB IT LI SE**

(56) Entgegenhaltungen :
**GB-A- 883 650**
**US-A- 3 105 845**

(73) Patentinhaber : **BASF Aktiengesellschaft**
**Carl-Bosch-Strasse 38**
**D-6700 Ludwigshafen (DE)**

(72) Erfinder : **Neumann, Peter, Dr.**
**Franz-Schubert-Strasse 1**
**D-6908 Wiesloch (DE)**
Erfinder : **Nissen, Dietmar, Dr.**
**Ziegelhäuser Landstrasse 31**
**D-6900 Heidelberg (DE)**
Erfinder : **Ohlinger, Rainer, Dr.**
**Ziegelhäuser Landstrasse 31**
**D-6900 Heidelberg (DE)**
Erfinder : **Werner, Frank, Dr.**
**Erschigweg 9**
**D-6730 Neustadt (DE)**

**EP 0 125 591 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Aromatische Diisocyanate, wie z. B. 2,4- und 2,6-Toluylendiisocyanat, 1,5-Naphthylen-diisocyanat, 4,4'-Diphenylmethan-diisocyanat und Mischungen aus 4,4'-, 2,4'- und 2,2'-Diphenylmethan-diisocyanten und Polyphenylpolymethylen-polyisocyanaten, aliphatische Diisocyanate, wie z. B. Hexamethylen-diisocyanat-1,6 und cycloaliphatische Diisocyante, wie z. B. 3-Isocyanatomethyl-3,5,5-trimethylcyclohexylisocyanat, sind bekannte Handelsprodukte, die vorzugsweise zur Herstellung von Polyurethankunststoffen Verwendung finden. Die Polyisocyanate werden üblicherweise aus den entsprechenden Aminoverbindungen durch Phosgenierung und anschließende thermische Spaltung der intermediär gebildeten Carbamidsäurechloride hergestellt. Zahlreiche organische Mono- und Polyisocyanate werden beispielsweise beschrieben in Annalen der Chemie 562 (1949), Seiten 75 ff.

Tri- oder tetraalkylsubstituierte aromatische Diisocyanate und Diisothiocyanate, bei denen die —NCO— bzw. —NCS-Gruppen in meta- oder para-Stellung zueinander stehen, sind bekannt aus der US-A-3 105 845. Die Alkylreste können hierbei 1 bis 18 Kohlenstoffatome besitzen und linear oder verzweigt sein. Die beispielhaft genannten Diisocyanate besitzen üblicherweise niedermolekulare Alkylreste, wie die Methyl- oder Ethylgruppe, und, aufgrund der symmetrischen Struktur, Isocyanatgruppen gleicher Reaktivität.

Die Aufgabe der vorliegenden Erfindung bestand darin, einkernige aromatische Diisocyanate zu entwickeln, deren Reaktivität im Vergleich zu m-Phenylendiisocyanat vermindert ist und deren Isocyanatgruppen gegebenenfalls eine unterschiedliche Reaktivität aufweisen.

Gelöst wurde diese Aufgabe durch trialkylsubstituierte aromatische Diisocyanate der Formeln

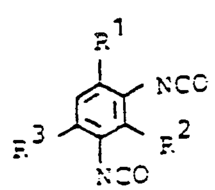 ,

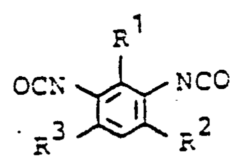 und/oder

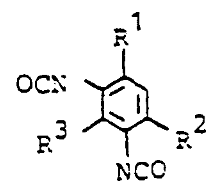 ,

(I) (II) (III)

in denen bedeuten :

$R^1$ eine in α-, β- oder γ-Stellung zum aromatischen Kern verzweigte Alkylgruppe mit 4 bis 12 Kohlenstoffatomen,

$R^2$ und $R^3$ gleiche oder verschiedene, lineare oder verzweigte Alkylgruppen mit 1 bis 4 Kohlenstoffatomen.

Durch Einführung mindestens eines orthoständig zur NCO-Gruppe gebundenen, sterisch anspruchsvollen verzweigtkettigen Alkylsubstituenten wird die Reaktivität der aromatischen Diisocyanate vermindert. Außerdem kann jedoch durch die Art und Stellung der drei Alkylgruppen im Diisocyanat die Reaktivität der zwei Isocyanatgruppen gezielt eingestellt und in vorteilhafter Weise den technischen Erfordernissen angepaßt werden. Ein Teil der erfindungsgemäßen trialkylsubstituierten aromatischen Diisocyanate besitzt somit Isocyanatgruppen unterschiedlicher Reaktivität, die für eine Reihe von Anwendungen von erheblicher Bedeutung ist.

Besonders bewährt haben sich und daher bevorzugt sind trialkylsubstituierte aromatische Diisocyanate der Formeln (I), (II) und/oder (III), in denen

$R^1$ eine in α-, β- oder γ-Stellung zum aromatischen Kern verzweigte Alkylgruppe mit 4 bis 8 Kohlenstoffatomen, insbesondere 4 bis 6 Kohlenstoffatomen,

$R^2$ und $R^3$ gleiche oder verschiedene, lineare oder verzweigte Alkylgruppen mit 1 bis 4 Kohlenstoffatomen

bedeuten.

Für den Alkylrest $R^1$ seien beispielhaft genannt :

$-CH_2-CH(CH_3)_2$, $-CH(CH_3)-C_2H_5$, $-C(CH_3)_3$,

$-CH(CH_3)-C_3H_7$, $-CH(CH_3)-CH(CH_3)_2$, $-CH(C_2H_5)_2$, $-CH_2-CH(CH_3)-C_2H_5$,

$-CH_2-C(CH_3)_3$, $-(CH_2)_2-CH(CH_3)_2$, $-CH(CH_3)-C_4H_9$, $-CH(CH_3)-CH(CH_3)-C_2H_5$,

$-C(CH_3)_2-C_3H_7$, $-CH(C_2H_5)-C_3H_7$, $-CH_2-CH(CH_3)-C_3H_7$, $-(CH_2)_2-C(CH_3)_3$,

$-CH_2-C(CH_3)_2-C_2H_5$,

$-CH(CH_3)-C_5H_{11}$, $-CH(CH_3)-CH(CH_3)-C_3H_7$, $-CH(CH_3)-CH(CH_3)-CH(CH_3)_2$, $-C(CH_3)_2-C_4H_9$, $-CH(C_2H_5)-C_4H_9$, $-CH(C_3H_7)_2$, $-C(CH_3)(C_2H_5)-C_3H_7$, $-C(C_2H_5)_3$, $-CH_2-CH(CH_3)-C_4H_9$, $-CH_2-CH_2-C(CH_3)_2-C_2H_5$, $-CH_2-C(CH_3)_2-C_3H_7$, $-CH_2-CH(C_2H_5)-C_3H_7$,

$-CH(CH_3)-C_6H_{13}$, $-CH(CH_3)-CH(CH_3)-C_4H_9$, $-CH(CH_3)-CH(CH_3)-CH_2-CH(CH_3)_2$, $-CH(CH_3)-CH(CH_3)-CH(CH_3)-C_2H_5$, $-CH(CH_3)-C(CH_3)_2-C_3H_7$, $-C(CH_3)_2-C_5H_{11}$, $-CH_2-CH(CH_3)-C_5H_{11}$, $-CH_2-C(CH_3)_2-C_4H_9$, $-CH_2-CH_2-C(CH_3)_2-C_3H_7$, $-CH(C_2H_5)-C_5H_{11}$, $-CH(C_3H_7)-C_4H_9$, $-C(CH_3)(C_2H_5)-C_4H_9$, $-C(C_2H_5)_2-C_3H_7$, $-C(CH_3)_2-CH_2-C(CH_3)_3$, $-CH_2-C(C_2H_5)_3$,

$-CH(CH_3)-C_7H_{13}$, $-CH(CH_3)-CH(CH_3)-C_5H_{11}$, $-CH(CH_3)-CH(CH_3)-CH_2-C(CH_3)_3$, $-CH(CH_3)-CH(CH_3)-CH(CH_3)-C_3H_7$, $-CH(CH_3)-C(CH_3)_2-C_4H_9$, $-C(CH_3)_2-C_6H_{13}$, $-CH_2-CH(CH_3)-C_6H_{13}$, $-CH_2-C(CH_3)_2-C_5H_{11}$, $-CH_2-CH_2-C(CH_3)_2-C_4H_9$, $-CH(C_2H_5)-C_6H_{13}$, $-CH(C_3H_7)-C_5H_{11}$, $-C(CH_3)(C_2H_5)-C_5H_{11}$, $-C(C_2H_5)_2-C_4H_9$, $-C(CH_3)_2-C(CH_3)_2-C_3H_7$, $-CH_2-C(C_2H_5)_2-C_3H_7$, $-CH_2-CH_2-C(C_2H_5)_3$,

$-CH(CH_3)-C_8H_{17}$, $-CH(CH_3)-CH(CH_3)-C_6H_{13}$, $-CH(CH_3)-CH(CH_3)-CH_2-C(CH_3)_2-C_2H_5$, $-CH(CH_3)-CH(CH_3)-CH(CH_3)-C_4H_9$,

$-CH(CH_3)-C(CH_3)_2-C_5H_{11}$, $-C(CH_3)_2-C_7H_{15}$, $-CH_2-CH(CH_3)-C_7H_{17}$, $-CH_2-C(CH_3)_2-C_6H_{13}$, $-CH_2-CH_2-C(CH_3)_2-C_5H_{11}$, $-CH(C_2H_5)-C_7H_{15}$, $-CH(C_3H_7)-C_6H_{13}$, $-C(CH_3)(C_2H_5)-C_6H_{13}$, $-C(C_2H_5)_2-C_5H_{11}$, $-C(CH_3)_2-C(CH_3)_2-C_4H_9$, $-CH_2-C(C_2H_5)_2-C_4H_9$, $-CH_2-CH_2-C(C_2H_5)_2-C_3H_7$,

$-CH(CH_3)-C_9H_{17}$, $-CH(CH_3)-CH(CH_3)-C_7H_{15}$, $-CH(CH_3)-CH(CH_3)-CH_2-C(CH_3)_2-C_3H_7$, $-CH(CH_3)-CH(CH_3)-CH(CH_3)-C_5H_{11}$, $-CH(CH_3)-C(CH_3)_2-C_6H_{13}$, $-C(CH_3)_2-C_8H_{17}$, $-CH_2-CH(CH_3)-C_8H_{17}$, $-CH_2-C(CH_3)_2-C_7H_{15}$, $-CH_2-CH_2-C(CH_3)_2-C_6H_{11}$, $-CH(C_2H_5)-C_8H_{17}$, $-CH(C_3H_7)-C_7H_{15}$, $-C(CH_3)(C_2H_5)-C_7H_{15}$, $-C(C_2H_5)_2-C_6H_{13}$, $-C(CH_3)_2-C(CH_3)_2-C_5H_{11}$, $-CH_2-C(C_2H_5)_2-C_5H_{11}$, $-CH_2-CH_2-C(C_2H_5)_2-C_4H_9$, $-CH_2-C(C_3H_7)_3$,

$-CH(CH_3)-C_{10}H_{21}$, $-CH(CH_3)-CH(CH_3)-C_8H_{17}$, $-CH(CH_3)-CH(CH_3)-CH_2-C(CH_3)_2-C_4H_9$, $-CH(CH_3)-CH(CH_3)-CH(CH_3)-C_6H_{13}$, $-CH(CH_3)-C(CH_3)_2-C_7H_{15}$, $-CH_2-C(CH_3)_2-C_8H_{17}$, $-CH_2-CH(CH_3)-C_9H_{19}$, $-CH_2-C(CH_3)_2-C_8H_{17}$, $-(CH_2)_2-C(CH_3)_2-C_7H_{15}$, $-CH(C_2H_5)-C_9H_{19}$, $-CH(C_3H_7)-C_8H_{17}$, $-C(CH_3)(C_2H_5)-C_8H_{17}$, $-C(C_2H_5)_2-C_7H_{15}$, $-C(CH_3)_2-C(CH_3)_2-C_6H_{13}$, $-C(C_2H_5)(C_3H_7)-C_6H_{13}$, $-C(C_3H_7)_2-C_5H_{11}$, $-C(C_4H_9)_2-C_3H_7$, $-CH_2-C(C_2H_5)_2-C_6H_{13}$, $-C(CH_3)_2-CH_2-C(CH_3)_2-CH_2-C(CH_3)_3$.

Als Alkylreste $R^2$ und $R^3$ kommen beispielsweise folgende Reste in Betracht :

$-CH_3$, $-C_2H_5$, $-n-C_3H_7$, $-CH(CH_3)_2$, $-n-C_4H_9$

Als insbesonders bevorzugte aromatische Diisocyanate sind beispielsweise Verbindungen der Formeln I, II und/oder III zu nennen, in denen bedeuten :

$R^1$ gleich $-CH(CH_3)-C_2H_5$, $-CH_2CH(CH_3)_2$, $-C(CH_3)_3$, $-CH(CH_3)-C_3H_7$, $-CH(CH_3)-CH(CH_3)_2$, $-CH(C_2H_5)_2$, $-CH_2-CH(CH_3)C_2H_5$, $-CH_2-C(CH_3)_3$, $-(CH_2)_2-CH(CH_3)_2$, $-C(CH_3)_2-C_2H_5$, $-CH(CH_3)-C_4H_9$, $-CH(CH_3)-CH(CH_3)-C_2H_5$, $-C(CH_3)_2-C_3H_7$, $-CH(C_2H_5)-C_3H_7$, $-CH_2-CH(CH_3)-C_3H_7$, $-(CH_2)_2-C(CH_3)_3$, $-CH_2-C(CH_3)_2-C_2H_5$.

$R^2 = R^3 = -CH_3$; $R^2 = -CH_3$, $R^3 = -C_2H_5$; $R^2 = -C_2H_5$, $R^3 = -CH_3$; $R^2 = -CH_3$, $R^3 = -C_3H_7$; $R^2 = -C_3H_7$, $R^3 = -CH_3$; $R^2 = -CH_3$, $R^3 = -CH(CH_3)_2$; $R^2 = -CH(CH_3)_2$, $R^3 = -CH_3$; $R^2 = -CH_3$; $R^3 = -n\text{-}C_4H_9$; $R^2 = -n\text{-}C_4H_9$, $R^3 = -CH_3$; $R^2 = -CH_3$; $R^3 = -CH(CH_3)-C_2H_5$; $R^2 = -CH(CH_3)-C_2H_5$, $R^3 = -CH_3$; $R^2 = -CH_3$, $R^3 = -CH_2-CH(CH_3)_2$; $R^2 = -CH_2-CH(CH_3)_2$, $R^3 = -CH_3$; $R^2 = -CH_3$, $R^3 = -C(CH_3)_3$; $R^2 = -C(CH_3)_3$, $R^3 = -CH_3$;

$R^2 = R^3 = -C_2H_5$; $R^2 = -C_2H_5$, $R^3 = -n\text{-}C_3H_7$; $R^2 = -n\text{-}C_3H_7$, $R^3 = -C_2H_5$; $R^2 = -C_2H_5$, $R^3 = -CH(CH_3)_2$; $R^2 = -CH(CH_3)_2$, $R^3 = -C_2H_5$; $R^2 = -C_2H_5$, $R^3 = -n\text{-}C_4H_9$; $R^2 = -n\text{-}C_4H_9$, $R^3 = -C_2H_5$; $R^2 = -C_2H_5$, $R^3 = -CH(CH_3)-C_2H_5$; $R^2 = -CH(CH_3)-C_2H_5$, $R^3 = -C_2H_5$; $R^2 = -C_2H_5$, $R^3 = -CH_2-CH(CH_3)_2$; $R^2 = -CH_2-CH(CH_3)_2$, $R^3 = -C_2H_5$; $R^2 = -C_2H_5$, $R^3 = -C(CH_3)_3$; $R^2 = -C(CH_3)_3$, $R^3 = -C_2H_5$;

$R^2 = R^3 = -n\text{-}C_3H_7$, $R^2 = -n\text{-}C_3H_7$, $R^3 = -CH(CH_3)_2$; $R^2 = -CH(CH_3)_2$, $R^3 = -n\text{-}C_3H_7$; $R^2 = -n\text{-}C_3H_7$, $R^3 = -n\text{-}C_4H_9$; $R^2 = -n\text{-}C_4H_9$, $R^3 = -n\text{-}C_3H_7$; $R^2 = -n\text{-}C_3H_7$, $R^3 = -CH(CH_3)-C_2H_5$; $R^2 = -CH(CH_3)-C_2H_5$, $R^3 = -n\text{-}C_3H_7$; $R^2 = -n\text{-}C_3H_7$, $R^3 = -CH_2-CH(CH_3)_2$; $R^2 = -CH_2-CH(CH_3)_2$, $R^3 = -n\text{-}C_3H_7$; $R^2 = -n\text{-}C_3H_7$, $R^3 = -C(CH_3)_3$; $R^2 = -C(CH_3)_3$, $R^3 = -n\text{-}C_3H_7$,

$R^2 = R^3 = -CH(CH_3)_2$; $R^2 = -CH(CH_3)_2$, $R^3 = -n\text{-}C_4H_9$; $R^2 = -n\text{-}C_4H_9$, $R^3 = -CH(CH_3)_2$; $R^2 = -CH(CH_3)_2$, $R^3 = -CH(CH_3)-C_2H_5$; $R^2 = -CH(CH_3)-C_2H_5$, $R^3 = -CH(CH_3)_2$; $R^2 = -CH(CH_3)_2$, $R^3 = -C(CH_3)_3$; $R^2 = -C(CH_3)_3$, $R^3 = -CH(CH_3)_2$;

$R^2 = R^3 = -n\text{-}C_4H_9$; $R^2 = -n\text{-}C_4H_9$, $R^3 = -CH(CH_3)-C_2H_5$; $R^2 = -CH(CH_3)-C_2H_5$, $R^3 = -n\text{-}C_4H_9$; $R^2 = -n\text{-}C_4H_9$, $R^3 = -CH_2-CH(CH_3)_2$; $R^2 = -CH_2-CH(CH_3)_2$, $R^3 = -n\text{-}C_4H_9$; $R^2 = -n\text{-}C_4H_9$, $R^3 = -C(CH_3)_3$; $R^2 = -C(CH_3)_3$, $R^3 = -n\text{-}C_4H_9$;
$R^2 = R^3 = -CH(CH_3)-C_2H_5$, $R^2 = -CH(CH_3)-C_2H_5$, $R^3 = -CH_2-CH(CH_3)_2$; $R^2 = -CH_2-CH(CH_3)_2$, $R^3 = -CH(CH_3)-C_2H_5$; $R^2 = -CH(CH_3)-C_2H_5$, $R^3 = -C(CH_3)_3$; $R^2 = -C(CH_3)_3$, $R^2 = -CH(CH_3)-C_2H_5$;

$R^2 = R^3 = -CH_2-CH(CH_3)_2$; $R^2 = -CH_2-CH(CH_3)_2$, $R^3 = -C(CH_3)_3$; $R^2 = -C(CH_3)_3$, $R^3 = -CH_2-CH(CH_3)_2$;

$R^2 = R^3 = -C(CH_3)_3$.

Die Herstellung der erfindungsgemäß einzusetzenden trialkylsubstitierten aromatischen Diamingemische erfolgt beispielsweise durch Nitrierung der entsprechenden Trialkylbenzole, Hydrierung oder Reduktion der enthaltenen Dinitroverbindung und anschließende überführung der erhaltenen trialkylsubstituierten aromatischen Diamine in die erfindungsgemäßen Diisocyanate. Die als Ausgangsstoffe verwendeten Trialkylbenzole lassen sich ausgehend von leicht zugänglichen Mono- oder 1,3-Dialkylbenzolen nach den beispielsweise in Rodd's Chemistry of Carbon Compounds, Band III/A, S. 102 ff (1. Auflage, Elsevier Publishing Comp., 1954) bzw. S. 156 ff. (2. Auflage, Elesevier Scientific Publishing Comp., 1983), J. Mathieu und J. Weill-Raynal, Formation of C-C-Bonds, Band II, S. 280 ff (Georg Thieme Publishers, Stuttgart, 1975) oder Methodicum Chimicum, Band 4, Grundgerüste, S. 171 ff (Georg Thieme Verlag, Stuttgart, New York 1980) beschriebenen Methoden herstellen. Von den dort genannten Verfahren ist aus wirtschaftlichen Gründen im allgemeinen die Alkylierung eines Mono- oder 1,3-Dialkylbenzols mit einem Alkylhalogenid, Alken oder Alkohol bevorzugt. Anstelle der reinen 1,3,5-Trialkylbenzole können für die weitere Umsetzung auch Gemische solcher Verbindungen verwendet werden, wie sie bei diesen Alkylierungsreaktionen anfallen.

Die Trialkylbenzole werden in an sich bekannter Weise zu einem Gemisch isomerer Dinitroverbindungen nitriert. Geeignete Verfahren sind beispielsweise Methoden der organischen Chemie, Houben-Weyl, Band X/1, S. 32 ff (Georg Thieme Verlag, Stuttgart, 1971) zu entnehmen. Die Reduktion der

Nitrogruppen zu den entsprechenden aromatischen gebundenen Aminogruppen kann durch katalytische Hydrierung, beispielsweise unter Verwendung von Raney-Nickel, Platin oder Palladium als Katalysator, erfolgen. Sie kann selbstverständlich auch nach anderen, an sich bekannten Reduktionsmethoden unter Verwendung von beispielsweise Eisen, Zink, Zinn oder auch Hydrazin als Reduktionsmittel durchgeführt werden. Das nach Entfernen des Katalysators und des zur Reduktion angewandten Lösungsmittels anfallende Diamingemisch läßt sich ohne weitere Reinigung oder, wenn auf die Herstellung besonders gereinigter Verfahrensprodukte Wert gelegt wird, nach Kristallisation oder Destillation zu den erfindungsgemäßen trialkylsubstituierten aromatischen Diisocyanaten umsetzen.

Die erhaltenen Trialkyl-diaminobenzole können direkt oder als Salze, vorzugsweise Hydrochloride, in Lösungsmitteln phosgeniert werden. Geeignete Lösungsmittel sind beispielsweise Toluol, Xylol, Chlorbenzol oder Dichlorbenzol. Die Trialkyl-diaminobenzole oder eine Suspension der entsprechenden Salze wird danach bei Temperaturen von ungefähr 0 °C bis 100 °C, vorzugsweise 0 bis 50 °C mit überschüssigem Phosgen zur Reaktion gebracht und das intermediär gebildete Carbamidsäurechlorid bei Temperaturen von 80 bis 180 °C vorzugsweise 120 bis 160 °C in das Trialkyl-diisocyanato-benzol gespalten. Das gasförmige oder flüssige Phosgen wird hierbei der Reaktionsmischung mit einer solchen Geschwindigkeit zugeführt, daß die austretenden Gase überwiegend aus Chlorwasserstoff bestehen.

Nach beendeter Phosgenierung und Spaltung wird das Lösungsmittel, vorzugsweise unter vermindertem Druck, beispielsweise von 100 bis 10 mbar abdestilliert. Gegebenenfalls kann es auch vorteilhaft sein, den Chlorwasserstoff und evtl. vorliegendes überschüssiges Phosgen mit Hilfe von Stickstoff oder einem anderen Inertgas aus der Diisocyanatlösung auszutreiben, bevor das Lösungsmittel abdestilliert wird.

Die erhaltenen rohen Trialkyl-diisocyanato-benzole oder deren Isomerengemische können durch Destillation unter vermindertem Druck getrennt und gereinigt werden.

Die erfindungsgemäßen Trialkyl-diisocyanato-benzole können ferner durch thermische Spaltung der entsprechenden Diurethane in der Gas- oder Flüssigphase, gegebenenfalls in Gegenwart von Katalysatoren hergestellt werden, wobei die Diurethane zweckmäßigerweise nach dem Verfahren der EP-OS 18 583 (US-PS 4 278 805) durch Umsetzung von Carbamidsäureester mit den Trialkyl-diamino-benzolen in Gegenwart von Alkoholen und gegebenenfalls Harnstoff erhalten werden.

Die neuen trialkylsubstituierten aromatischen Diisocyanate sind wertvolle Ausgangsmaterialien für Pflanzenschutzmittel und Kunststoffe. Die Produkte eignen sich insbesondere zur Herstellung von Polyurethanschaumstoffen, -elastomeren, -klebstoffen, -lacken, -beschichtungsmitteln und -dichtungsmitteln.

Die folgenden Beispiele sollen die Erfindung näher erläutern, ohne sie jedoch zu beschränken.

## Beispiel 1

a) Zu 402 g 90 gew.%iger Schwefelsäure gab man bei 0 bis 5 °C 325 g 1,3-Dimethyl-5-tert.-butylbenzol. Anschließend ließ man unter intensivem Rühren und weiterer Kühlung ein Gemisch aus 402 g 90-gew.%iger Schwefelsäure und 270 g 98 gew.%iger Salpetersäure mit einer solchen Geschwindigkeit hinzutropfen, daß die Temperatur der Reaktionsmischung 20 °C nicht überstieg. Danach rührte man 1 Stunde bei 20 bis 25 °C und 1 Stunde bei 35 °C bis maximal 40 °C nach, fällte das Reaktionsgemisch mit ca. 5 l Eiswasser aus und saugte die Fällung nach Abkühlen auf Raumtemperatur ab. Man erhielt 590 g feuchtes Nitrierungsprodukt vom Schmelzpunkt 47 bis 50 °C. Es konnte direkt weiterverarbeitet oder wie folgt gereinigt werden :

Man löste in etwa der 8- bis 9-fachen Menge Methanol bei 45 °C, neutralisierte mit 10 gew.%iger Sodalösung und fällte das Nitrierungsprodukt mit Wasser aus. Nach Abkühlen auf ca. 15 °C wurde die Fällung abgesaugt und mit Wasser gewaschen. Man erhielt ca. 480 g feuchtes 2,4- bzw. 2,6-Dinitro-1,3-dimethyl-5-tert.-butylbenzol-Isomerengemisch vom Schmp. 57 bis 59 °C.

b) 480 g des nach a) erhaltenen Isomerengemisches wurden in 1 l Isobutanol/40 g Raney-Nickel bei 80 °C und 50 bar hydriert. Nach Abtrennung des Katalysators wurde zunächst das Lösungsmittel bei 10 bis 20 mbar und der Rückstand im Hochvakuum destilliert. Man erhielt 265 g 2,4-bzw. 2,6-Diamino-1,3-dimethyl-5-tert.-butylbenzol-Isomerengemisch vom Siedepunkt 142 bis 152 °C (1,2 bis 1,3 mbar), das beim Abkühlen erstarrte.

c) In 600 ml o-Dichlorbenzol wurden bei 0 °C 240 g Phosgen eingegast. Zur Phosgenlösung ließ man gleichfalls bei 0 °C eine Lösung von 57,3 g 2,4-bzw. 2,6-Diamino-1,3-dimethyl-5-tert.-butylbenzol-Isomerengemisch in 240 ml o-Dichlorbenzol zutropfen. Anschließend erwärmte man das Reaktionsgemisch unter gutem Rühren langsam auf 130 °C und leitete bei dieser Temperatur weiter Phosgen ein (ca. 1,5 Stunden). Man ließ das Reaktionsgemisch auf Raumtemperatur abkühlen und trennte das überschüssige Phosgen durch Einleiten von Stickstoff ab. Danach wurde zunächst das Lösungsmittel bei 10 bis 20 mbar abgetrennt und darauf der Rückstand im Hochvakuum destilliert. Man erhielt 61,7 g 2,4- bzw. 2,6-Diisocyanato-1,3-dimethyl-5-tert.-butylbenzol-Isomerengemisch mit einem Siedepunkt von 105 bis 115 °C (0,25 mbar), Schmp. 88 bis 95 °C.

Analyse : $C_{14}H_{16}O_2N_2$ (Molekulargewicht : 244, massenspektrometrisch)

| | | | | |
|---|---|---|---|---|
| ber. : | C 68,83 | H 6,60 | O 13,10 | N 11,47 |
| gef. : | C 68,7 | H 6,5 | O 13,0 | N 11,7 |

Beispiel 2

a) Zu 213 g wasserfreiem Aluminiumchlorid ließ man langsam bei 0 bis 5 °C eine Mischung aus 371 g m-Xylol und 101,2 g 2-Methylbutanol-(2) zutropfen. Nach Beendigung des Zutropfens rührte man noch 5 Stunden bei 0 bis 10 °C nach und rührte danach das Reaktionsgemisch vorsichtig in ca. 5 l Eiswasser ein, trennte die organische Phase ab, wusch sie zweimal mit je 500 ml Wasser und fraktionierte anschließend unter vermindertem Druck. Man erhielt 154 g eines bei 111 bis 113 °C (29 mbar) siedenden Gemisches von 1,3-Dimethyl-5-(1,2-dimethyl-propyl)- und 1,3-Dimethyl-5-(1,1-dimethylpropyl)-benzol (Hauptkomponenten).

b) In ein Gemisch aus 80,4 g 90 gew.%iger Schwefelsäure und 70,4 g der nach a) erhaltenen 1,3-Dimethyl-5-(1,2- bzw. 1,1-dimethylpropyl)-benzolmischung ließ man bei 15 bis 20 °C ein Gemisch aus 80,4 g 90 gew.%iger Schwefelsäure und 59,6 g 98 %iger Salpetersäure zutropfen. Anschließend rührte man 1 Stunde bei 20 bis 25 °C und 1 Stunde bei 30 bis maximal 40 °C nach, fällte das Reaktionsgemisch mit ca. 500 ml Eiswasser aus und nahm das abgeschiedene Öl mit ca. 200 ml Toluol auf. Die organische Phase wurde abgetrennt, mit Wasser, verdünnter Sodalösung und wieder mit Wasser gewaschen. Nach Entfernung des Lösungsmittels unter vermindertem Druck erhielt man 81 g eines Gemisches aus 2,4- bzw. 2,6-Dinitro-1,3-dimethyl-5-(1,2-dimethylpropyl)- und 2,4- bzw. 2,6-Dinitro-1,3-dimethyl-5-(1,1-dimethylpropyl)-benzol (Hauptkomponenten), das direkt weiterverarbeitet werden konnte.

c) 50 g der nach b) erhaltenen rohen Trialkyl-dinitrobenzolmischung wurden in 140 ml Isobutanol/5 g Raney-Nickel bei 80 °C und 50 bar hydriert. Nach Abtrennung des Katalysators destillierte man das Lösungsmittel bei 10 bis 20 mbar und danach den Rückstand. Man erhielt 19 g eines bei 130 bis 134 °C siedenen Gemisches von 2,4- bzw. 2,6-Diamino-1,3-dimethyl-5-(1,2-dimethylpropyl)- und 2,4- bzw. 2,6-Diamino-1,3-dimethyl-5-(1,1-dimethylpropyl)-benzol.

d) In 200 ml o-Dichlorbenzol wurden bei 0 °C 80 g Phosgen eingegast. Zur Phosgenlösung ließ man gliechfalls bei 0 °C eine Lösung von 17,1 g des nach c) erhaltenen Diamino-trialkylbenzolgemisches in 80 ml o-Dichlorbenzol zutropfen. Anschließend erwärmte man unter gutem Rühren langsam auf 130 °C und leitete bei dieser Temperatur weiter Phosgen ein (ca. 1,5 Stunden). Man ließ das Reaktionsgemisch auf Raumtemperatur abkühlen und trennte das überschüssige Phosgen durch Einleiten von Stickstoff ab. Danach wurde zunächst das Lösungsmittel bei 10 bis 20 mbar und darauf der Rückstand im Hochvakuum destilliert. Man erhielt 16 g eines Gemisches von 2,4- bzw. 2,6-Diisocyanato-1,3-dimethyl-5-(1,2-dimethyl-propyl)- und 2,4- bzw. 2,6-Diisocyanato-1,3-dimethyl-5-(1,1-dimethylpropyl)-benzol vom Siedepunkt 140 bis 150 °C (1,2 bis 1,3 mbar).

Analyse : $C_{15}H_{18}N_2O_2$ (Molekulargewicht : 258, massenspektrometrisch)

| | | | | |
|---|---|---|---|---|
| ber. : | C 69,75 | H 7,02 | N 10,84 | O 12,39 |
| gef. : | C 69,8 | H 7,0 | N 11,0 | O 12,2 |

Beispiel 3

a) 1 kg eines analog den Angaben von Beispiel 1a) hergestellten rohen, wasserfeuchten 2,4- bzw. 2,6-Dinitro-1,3-dimethyl-5-tert.-butylbenzols wurden in 2 l Methanol bei 50 °C gelöst. Man ließ unter Rühren auf Raumtemperatur abkühlen, saugte den Niederschlag ab und behandelte ihn erneut in der beschriebenen Weise mit 600 ml Methanol. Nach dem Trocknen des Niederschlags bei 50 °C unter vermindertem Druck erhielt man 507 g 2,4-Dinitro-1,3-dimethyl-5-tert.-butylbenzol vom Schmelzpunkt : 65-66 °C.

b) 750 g der nach Beispiel 3a) erhaltenen Dinitroverbindung wurden in 2 l Isopropanol/50 g Raney-Nickel bei 100 °C und 50 bar hydriert und danach analog Beispiel 1b) aufgearbeitet. Man erhielt 523 g 2,4-Diamino-1,3-dimethyl-5-tert.-butylbenzol vom Siedepunkt 155-157 °C (0,4 mbar), das beim Abkühlen erstarrte (Schmelzpunkt 87 bis 90 °C).

c) In 300 ml o-Dichlorbenzol wurden bei 0 °C 128 g Phosgen eingegast. Zur Phosgenlösung ließ man gleichfalls bei 0 °C eine Lösung von 30,7 g 2,4-Diamino-1,3-dimethyl-5-tert.-butylbenzol in 120 ml o-Dichlorbenzol zutropfen. Anschließend verfuhr man wie in Beispiel 1c) beschrieben. Nach der Destillation erhielt man 28,4 g 2,4-Diisocyanato-1,3-dimethyl-5-tert.-butylbenzol mit einem Siedepunkt von 123 bis 125 °C (0,25 mbar), Schmelzpunkt 99 bis 101 °C.

Analyse : $C_{14}H_{16}O_2N_2$ (Molekulargewicht 244, massenspektrometrisch)

| | | | | |
|---|---|---|---|---|
| ber. : | C 68,83 | H 6,60 | O 13,10 | N 11,47 |
| gef. : | C 68,6 | H 6,6 | O 12,9 | N 11,6 |

Beispiel 4

a) In eine auf — 5 °C gekühlte Mischung aus 38 ml konz. Schwefelsäure und 25 g 1-Methyl-3,5-di-tert.-butylbenzol ließ man ein Gemisch aus 25 ml Salpetersäure (98 %) und 25 ml konz. Schwefelsäure in der Weise hinzutropfen, daß die Temperatur maximal 0 °C betrug. Nach Beendigung des Zutropfens erwärmte man langsam auf Raumtemperatur (Nachreaktion) und anschließend eine Stunde auf 35 °C. Das Reaktionsgemisch wurde auf 300 g Eis ausgetragen, der Niederschlag abgesaugt, mit Wasser gewaschen und an der Luft getrocknet. Nach Umkristallisation aus Ethanol erhielt man 25,1 g 2,6-Dinitro-1-methyl-3,5-di-tert.-butylbenzol vom Schmelzpunkt 174 bis 175 °C.

b) 57 g der nach Beispiel 4a) erhaltenen Dinitroverbindung wurden in 1 l Isobutanol/15 g Raney-Nickel bei 40 °C und 20 bar hydriert. Nach der Aufarbeitung analog Beispiel 1b) erhielt man 37 g 2,6-Diamino-1-methyl-3,5-di-tert.-butylbenzol vom Siedepunkt 162 bis 165 °C (0,35 mbar).

c) In 300 ml o-Dichlorbenzol wurden bei 0 °C 120 g Phosgen eingegast. Zur Phosgenlösung ließ man bei 0 °C eine Lösung von 35,1 g des nach Beispiel 3b) erhaltenen Diamins in 120 ml o-Dichlorbenzol zutropfen. Anschließend verfuhr man wie in Beispiel 1c) beschrieben. Nach der Destillation erhielt man 37,1 g 2,6-Diisocyanato-1-methyl-3,5-di-tert.-butylbenzol vom Siedepunkt 139 bis 140 °C (0,25 mbar).

Analyse : $C_{17}H_{22}O_2N_2$ (Molekulargewicht 286, massenspektrometrisch)

| | | | | |
|---|---|---|---|---|
| ber. : | C 71,30 | H 7,74 | O 11,17 | N 9,78 |
| gef. : | C 71,0 | H 7,7 | O 11,4 | N 9,8 |

**Patentansprüche**

1. Aromatische Diisocyanate der Formeln

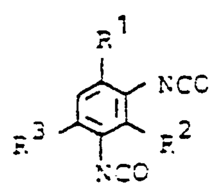 ,

(I)

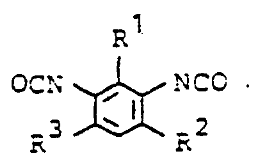 und/oder

(II)

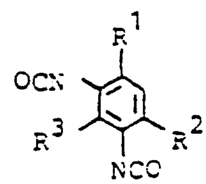 ,

(III)

in denen bedeuten :

$R^1$ eine in α-, β- oder γ-Stellung zum aromatischen Kern verzweigte Alkylgruppe mit 4 bis 12 Kohlenstoffatomen,

$R^2$ und $R^3$ gleiche oder verschiedene, lineare oder verzweigte Alkylgruppen mit 1 bis 4 Kohlenstoffatomen.

2. 1,3-Dimethyl-5-tert.-butyl-2,4-diisocyanatobenzol und/oder 1,3-Dimethyl-5-tert.-butyl-2,6-diisocyanatobenzol.

3. 1,3-Dimethyl-5-(1,2-dimethylpropyl)-2,4-diisocyanatobenzol, 1,3-Dimethyl-5-(1,2-dimethylpropyl)-2,6-diisocyanatobenzol, 1,3-Dimethyl-5-(1,1-dimethylpropyl)-2,4-diisocyanatobenzol und/oder 1,3-Dimethyl-5-(1,1-dimethylpropyl)-2,6-diisocyanatobenzol.

4. Verfahren zur Herstellung von aromatischen Diisocyanaten der Formeln (I), (II) und/oder (III), dadurch gekennzeichnet, daß man die entsprechenden trialkylsubstituierten aromatischen Diamine phosgeniert und die hierbei entstehenden Carbamidsäurechloride thermisch spaltet.

5. Verwendung von trialkylsubstituierten aromatischen Diisocyanaten der Formeln (I), (II) und/oder (III) zur Herstellung von Polyurethan-Kunststoffen oder Pflanzenschutzmitteln.

**Claims**

1. Aromatic diisocyanates of the formulae

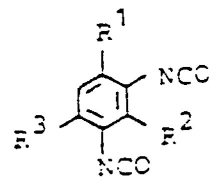, 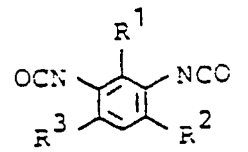 and/or 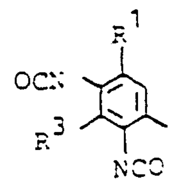,

(I) (II) (III)

where

$R^1$ is alkyl of 4 to 12 carbon atoms which is branched at the α-, β- or γ-position on the aromatic nucleus, and

$R^2$ and $R^3$ are each identical or different linear or branched alkyls of 1 to 4 carbon atoms.

2. 1,3-Dimethyl-5-tert.-butyl-2,4-diisocyanatobenzene and/or 1,3-dimethyl-5-tert.-butyl-2,6-diisocyanatobenzene.

3. 1,3-Dimethyl-5-(1,2-dimethylpropyl)-2,4-diisocyanatobenzene, 1,3-dimethyl-5-(1,2-dimethylpropyl)-2,6-diisocyanatobenzene, 1,3-dimethyl-5-(1,1-dimethylpropyl)-2,4-diisocyanatobenzene and/or 1,3-dimethyl-5-(1,1-dimethylpropyl)-2,6-diisocyanatobenzene.

4. A process for the preparation of aromatic diisocyanates of the formulae (I), (II) and/or (III), wherein the corresponding trialkyl-substituted aromatic diamines are phosgenated, and the resulting carbamyl chlorides are thermally cleaved.

5. The use of trialkyl-substituted aromatic diisocyanates of the formulae (I), (II) and/or (III) for the preparation of polyurethane plastics or crop protection agents.

**Revendications**

1. Diisocyanates aromatiques des formules

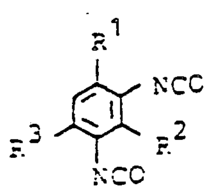, 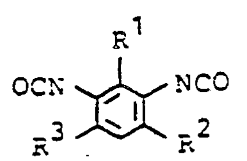 et/ou 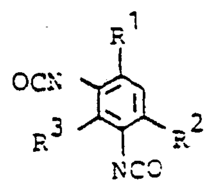,

(I) (II) (III)

dans lesquelles

$R^1$ représente un groupe alkyle en $C_4$ à $C_{12}$, ramifié en position α, β ou γ par rapport au noyau aromatique et

$R^2$ et $R^3$, qui peuvent être identiques ou différents, désignent chacun un radical alkyle à chaîne droite ou ramifiée en $C_1$ à $C_4$.

2. Le 1,3-diméthyl-5-tert.-butyl-2,4-diisocyanato-benzène et (ou) le 1,3-diméthyl-5-tert.-butyl-2,6-diisocyanato-benzène.

3. Le 1,3-diméthyl-5-(1,2-diméthylpropyl)-2,4-diisocyanato-benzène, le 1,3-diméthyl-5-(1,2-diméthylpropyl)-2,6-diisocyanato-benzène, le 1,3-diméthyl-5-(1,1-diméthylpropyl)-2,4-diisocyanato-benzène et (ou) le 1,3-diméthyl-5-(1,1-diméthylpropyl)-2,6-diisocyanato-benzène.

4. Procédé de préparation de diisocyanates aromatiques des formules (I), (II) et (III), caractérisé en ce que l'on soumet les diamines aromatiques trialkylsubstituées correspondantes à une phosgénation et les chlorures d'acide carbamique formés dans cette réaction à un clivage thermique.

5. Utilisation de diisocyanates aromatiques trialkylsubstitués des formules (I), (II) et (ou) (III) pour la préparation de matières plastiques polyuréthanniques et de produits phytosanitaires.